# EUROPEAN PATENT SPECIFICATION

(11) **EP 3 283 630 B1**
(45) Date of publication and mention of the grant of the patent: **26.07.2023**
(21) Application number: 15888914.7
(22) Date of filing: 18.06.2015
(51) Int. Cl.: C12N 15/11, C12N 15/10, C12Q 1/68, C12Q 1/6858, C12Q 1/6853

(54) **OLIGONUCLEOTIDE FRAGMENT, AND METHOD AS WELL AS APPLICATION OF SELECTIVE AMPLIFICTION OF VARIANT OF TARGET NUCLEIC ACID SEQUENCE USING THE SAME**
OLIGONUKLEOTIDFRAGMENT UND VERFAHREN SOWIE ANWENDUNG VON SELEKTIVER AMPLIFIKATION VON VARIANTEN EINER ZIELNUKLEINSÄURESEQUENZ DAMIT
FRAGMENT OLIGONUCLÉOTIDIQUE ET PROCÉDÉ AINSI QU'APPLICATION D'AMPLIFICATION SÉLECTIVE D'UN VARIANT DE SÉQUENCE D'ACIDE NUCLÉIQUE CIBLE L'UTILISANT

(30) Priority: 15.04.2015 CN 201510177952
(43) Date of publication of application: 21.02.2018
(73) Proprietor: Nuhigh Biotechnologies Co., Ltd, Suzhou, Jiangsu 215123 (CN)
(72) Inventor: BI, Wanli, Suzhou Jiangsu 215123 (CN); WANG, Zhifeng, Suzhou Jiangsu 215123 (CN); XU, Xin, Suzhou Jiangsu 215123 (CN)
(74) Representative: HGF
(86) International application number: PCT/CN2015/081818
(87) International publication number: WO 2016/165210

(56) References cited:
- EP-A1- 0 963 443
- WO-A1-2013/123220
- WO-A2-2011/100057
- CN-A- 1 580 277
- US-A1- 2003 073 101
- US-A1- 2007 264 653
- RAND KEITH N ET AL: "Headloop suppression PCR and its application to selective amplification of methylated DNA sequences", NUCLEIC ACIDS RESEARCH, OXFORD UNIVERSITY PRESS, GB, vol. 33, no. 14, 1 January 2005 (2005-01-01), pages e127-1, XP002515951, ISSN: 1362-4962, DOI: 10.1093/NAR/GNI120
- SOLINAS A ET AL: "Duplex Scorpion primers in SNP analysis and FRET applications", NUCLEIC ACIDS RESEARCH, INFORMATION RETRIEVAL LTD, vol. 29, no. 20, 15 October 2001 (2001-10-15), page E96, XP002318909, ISSN: 0305-1048, DOI: 10.1093/NAR/29.20.E96

## Description

The present application claims the priority of Chinese patent application No. 201510177952.5, filed on April 15, 2015, entitled "oligonucleotide fragment, and method as well as application of selective amplification of variant of target nucleic acid sequence using the same".

### Field of the invention

The present invention belongs to the field of molecular biology, and relates to a method for selective amplification of a variant of a target nucleic acid sequence, particularly to an oligonucleotide fragment, as well as a method for selective amplification of a variant of a target nucleic acid sequence using the same. The present invention further relates to application of a kit for detecting a nucleic acid variant, which is widely applied in fields such as nucleic acid amplification, tumor *in vitro* diagnosis, genotyping and the like.

### Background of the invention

An allele generally refers to a pair of genes which locate at the same position in a pair of homologous chromosomes and control relative characters, and it has polymorphism. Allele polymorphism analysis is used widely, including forensic identification, drug property analysis, genotyping, organ transplantation, and also including detection of a gene mutation in tumor cells under a background having a large quantity of wild-type genes. However, usually, tumor cells account for a rather low proportion compared to surrounding normal tissue cells, making it very difficult to detect the tumor cells, in particular, especially difficult to detect a single point mutation of tumor genes under a background having a large quantity of wild-type genes. In order to detect a small quantity of mutant-type variants of target nucleic acid sequences, currently, detection is generally performed by combination of a selective amplification method and a selective detection method. The selective detection method includes high-resolution melting curve, probe hybridization, TaqMan^{™} probe and other methods. The TaqMan^{™} probe method disclosed in U.S. patents (U.S. Pat. Nos. 5,210,015, 5,538,848 and 6,326,145) is a process in which a probe is specifically hybridized with a nucleic acid fragment to be detected, followed by cleavage by the DNA polymerase having 5' exonuclease activity, with the generated signal being used for indicating the presence of the target fragment to be detected. However, a very little proportion of the mutant-type variant would easily cause the signal drowned out by the signals generated from the wild-type variants which are similar with the those generated from the mutant-type variants of the target sequence to be detected; while the selective amplification method has high selectivity.

Therefore, allele selective amplification allows selective amplification and detection of a mutant-type variant of a target nucleic acid sequence. In a successful selective amplification system, only a mutant-type variant of a target nucleic acid sequence is amplified, while a wild-type variant of the target nucleic acid sequence is not amplified; or the amplification efficiency of the wild-type variant of the target nucleic acid sequence is far lower than that of the mutant-type variant of the target nucleic acid sequence. For the purpose of selective amplification, various methods have been developed, such as PCR clamping method, RFLP-PCR method, digital PCR method, coamplification PCR method at lower denaturation temperature, as described below.

The PCR clamping method is a method by inhibiting the amplification of a wild-type variant of a target nucleic acid sequence to achieve selective amplification of a target fragment to be detected. Methods using a peptide nucleic acid (PNA) or locked nucleic acid (LNA) have been disclosed in documents [Henrik et al., Nucleic Acid Research 21:5332-5336(1993)] and [Luo et al., Nucleic Acid Research Vol. 34, No 2 e12 (2006)], respectively. However, unlike the wild-type variant and mutant-type variant of the target nucleic acid sequence generally having only one base different from the target fragment to be detected, this method cannot perfectly inhibit amplification of these non-target fragments.

The RFLP-PCR method is a method which employs restriction endonuclease prior to or during a PCR reaction to remove wild-type variants similar with mutant-type variants of a target nucleic acid sequence, thereby achieving amplification and enrichment of the mutant-type variants of the target nucleic acid fragment. Based on this method, there are various variations, including a restriction site mutation-PCR (RSM-PCR) method, amplification based on primer ligation at a mutation site (APRIL-ATM), and the like. Such methods have advantages of simple design and low cost, and have good selectivity in some specific experiments; however, they rely on having a restriction endonuclease site near the mutation site, which have limited selectivity in specific applications.

Digital PCR is a method by diluting templates and increasing the number of PCR reactions to detect a small quantity of mutant-type variants of a target nucleic acid sequence [Vogelstein B, Kinzler KW. Digital PCR. Proc Natl Acad Sci USA 1999; 96: 9236-41]. In theory, when the nucleic acid templates are diluted that only one or no nucleic acid template is present in each PCR reaction, in this PCR reaction, either nothing is amplified, or a wild-type template or a mutant-type template is amplified. By combining with a corresponding detection method, detection of a small quantity of mutant-type variants can be achieved. In theory, such a method has an unlimited selectivity by increasing the number of PCR reactions. However, in an actual operation, the selectivity is not only limited by the fidelity of Taq DNA polymerase, but also is limited by the PCR number which can be performed simultaneously. It has been reported that a digital PCR-based method has a high selectivity, for example, the content as disclosed in [Bielas JH, Loeb LA. Quantification of random genomic mutations. Nat Methods 2005;2:285-90]. However, this method usually requires a specialized instrument and is performed by the aid of a chip technology, and it has a very complicated process and high cost.

Coamplification at lower denaturation temperature PCR (Cold-PCR) is a method by adjusting thermal cycle conditions of PCR reactions to enrich target fragments to be amplified. At a lower denaturation temperature (Tc), a mutant-type variant of a target nucleic acid sequence or a heterodimer formed from a mutant-type variant and a wild-type variant thereof is more easily denatured for further amplification and enrichment, as compared to a wild-type variant. Such a method can provide some selectivity, but such a selectivity is still insufficient, for example, the content as disclosed in [Li J, Wang L, Mamon H, Kulke MH, Berbeco R, Makrigiorgos GM. Replacing PCR with COLD-PCR enriches variant DNA sequences and redefines the sensitivity of genetic testing. Nat Med 2008; 14:579-84].

In a typical polymerase chain reaction PCR, the upstream primer and downstream primer are completely complementary to the two chains of a target gene, respectively. If there are one or more base mismatches at 3' terminal of a primer, it is possible to cause substantial reduction of the amplification efficiency. Some of such mismatches have a large influence while some have a small influence. In a system for detection of a single point mutation, the last base or the second last base at 3' terminal of a primer has been designed to be completely complementary and matched to the mutation site, thereby selectively amplifying the mutant gene while having very low amplification efficiency to normal non-mutant genes. Such a method is referred to as an allele specific amplification method (see U.S. Pat 5639611). Such a selective amplification method can achieve better selectivity by adjustment of reaction conditions of the system. In spite of this, the selectivity of allele specific PCR is far from enough in a variety of applications.

Regarding researches to increase the selectivity, an improved method is to additionally introduce a mutation site in a primer so as to increase the selectivity of a system (see U.S. Pat. No. 5137806); and another method is to use a chemically modified primer to increase the selectivity of the allele specific PCR method. For example, 2'-C, 4'-C modifications at specific positions of deoxyribose on certain nucleotides of a primer can increase the selectivity [Gaster, J. and Marx, A Chem. Eur.J.2005, 11:1861-1870]. In addition, there is a method of introduction of an unusual base to a primer to increase the selectivity of a system (see U.S. Pat No. 7408051), and a method by using a thermostable protein for mismatch binding to increase the specific selectivity of a system (see US 5877280).

In an allele specific PCR, the selectivity of a system is embodied on a difference value between Ct values of amplification. A template which is completely matched and a template which is not completely matched for primer amplification have different efficiency, and generate different Ct values. The larger the difference between Ct values thereof is, the higher the selectivity of the system will be. The resolution of such a difference is enough to a variety of applications, but it may be not enough while detection of a small quantity of mutant-type variants present in a large quantity of wild-type variants in a tumor tissue. Through a series of methods as described above, the resolution may be improved to a certain extent; however, the improvement is still limited in a specific application. Moreover, by using a primer mismatched at 3' terminal, once a wrong extension occurs, it corresponds to artificial introduction of a mutant-type variant, and such an artificially introduced mutant-type variant is apt to be amplified as a mutant-type variant in each round of subsequent cycles.

US20070264653A1 discloses a method of identifying a biological sample for methylation analysis. US20030073101A1 discloses methods for determining genotypes and haplotypes of genes, that is, to determine the nucleotide present at a selected polymorphic site in a target nucleic acid molecule. WO2013123220A1 discloses methods for identifying the presence of one or more target nucleotide sequences in a sample that involve a nuclease-ligation reaction. EP0963443A1 discloses the synthesis and use of mass-labeled compounds to specifically interact with biomolecular targets.

### Summary of the invention

The present invention is defined by the appended independent claims. Further optional features of the invention are defined in the dependent claims. The present invention provides an oligonucleotide fragment, which may be applied in selective amplification of a variant of a target nucleic acid sequence, in a kit for detection of a nucleic acid variant, and may be widely used in the fields like nucleic acid amplification, tumor *in vitro* diagnosis, genotyping, etc.

The oligonucleotide fragment provided in the present invention comprises a primer region at 3' terminal and a variant recognition region at 5' terminal thereof which are covalently linked, the variant recognition region being covalently linked with a nucleic acid double strand-stabilizing factor or comprising a modifying factor which cannot be hydrolyzed by nuclease, and the variant recognition region forming a hairpin structure with a sequence generated from extension of the primer region.

In a further aspect of the present invention, the modifying factor includes a base analogue, nucleic acid backbone, deoxyribose analogue, peptide nucleic acid or thiophosphate.

In a further aspect of the present invention, the nucleic acid double strand-stabilizing factor includes one of or a combination of more than one of a locked nucleic acid, peptide nucleic acid or DNA minor groove binder/analogue.

The present invention further provides a method for selective amplification of a variant of a target nucleic acid sequence using the oligonucleotide fragment as described above, the method comprising:
(1) hybridizing a primer region in the oligonucleotide fragment with the target nucleic acid sequence in a reaction system, to prepare a template for amplification;
(2) extending 3' terminal of the primer region by nucleic acid polymerase in an amplification system;
(3) when a variant in the sample to be detected being used as a template, a variant recognition region and the primer region of the oligonucleotide fragment extending to generate a sequence which cannot form a stable hairpin structure, thus an amplification reaction proceeding,
when a nucleic acid variant not to be detected in the sample to be detected being used as a template, the variant recognition region and the primer region of the oligonucleotide fragment extending to generate a sequence which forms a hairpin structure, thus the amplification reaction being inhibited.

In a further aspect of the present invention, a detection method which does not distinguish different nucleic acid variants is used in combination with a method including a dye method, hybridization probe detection method, hydrolysis probe detection method, Taqman probe method or molecular beacon method.

In a further aspect of the present invention, a method distinguishing different nucleic acid variants is used in combination with a method including sequencing or high-resolution melting curve; alternatively, a method like hybridization probe distinguishing different nucleic acid variants, hydrolysis probe distinguishing different nucleic acid variants, Taqman probe distinguishing different nucleic acid variants or molecular beacon distinguishing different nucleic acid variants, is used in combination.

In a further aspect of the present invention, the amplification system is a system which initiates a reaction with the increase of temperature.

In another aspect, the present invention further provides a kit for detection of a nucleic acid variant, which detects the nucleic acid variant by using any of the oligonucleotide fragments as described above.

In a further aspect of the present invention, the kit further comprises a reagent required for nucleic acid detection which includes a nucleic acid dye and/or probe.

In a further aspect of the present invention, the kit further comprises a kit amplification system, which comprises one or a combination of more of nucleic acid polymerases, divalent metal ions, nucleotides, salts, buffers or cofactors.

The present invention has the following advantageous effects.
1. Higher selectivity. The present invention has an excellent selectivity, and can detect trace amount of a nucleic acid variant to be detected in a large quantity of nucleic acid variants not to be detected. For traditional allele specific PCR method, it uses a primer having a mismatch at 3' terminal. Once a wrong extension occurs, it corresponds to artificial introduction of a mutant-type variant, and such an artificially introduced mutant-type variant will be amplified as a mutant-type variant in each of subsequent cycles; while the present invention does not have this problem. In the present invention, even though the variant not to be detected is amplified in a round of PCR reaction, the amplified product will still be apt to form a hairpin structure in the next round of the reaction such that efficient amplification cannot be achieved, thereby efficiently inhibiting the amplification efficiency of the variant not to be detected and achieving the selective amplification.
2. Simple primer design and convenient operation.
3. Wide application. The present invention may be widely applied to fields such as nucleic acid amplification, tumor *in vitro* diagnosis, genotyping, etc.

### Brief description of the drawings

In order to illustrate more clearly the examples of the present invention or technical solutions in the prior art, figures used in the examples or in the prior art will be simply introduced hereinafter. Apparently, the figures in the following description are only for some examples of the present invention, and for those skilled in the art, other figures may be obtained from these figures without creative work.
Fig. 1 is a schematic diagram of a hairpin structure.
Fig. 2 is a schematic diagram of the technical principle using an oligonucleotide fragment of the present invention to achieve selective amplification.
Fig. 3 is a schematic diagram of selective amplification results of EGFR G719S using the oligonucleotide fragment of the present invention.
Fig. 4 is a schematic diagram of selective amplification results of EGFR T790M using the oligonucleotide fragment of the present invention.
Fig. 5 is a schematic diagram of selective amplification results of a mixed template using the oligonucleotide fragment of the present invention.
Fig. 6 is a schematic diagram achieving selective amplification and selective detection using the oligonucleotide fragment of the present invention and a MGB probe.
Fig. 7 is a schematic diagram of amplification results using the oligonucleotide fragment of the present invention which does not comprise nucleic acid double strand-stabilizing factor.
Fig. 8 is a schematic diagram of amplification results using the oligonucleotide fragment of the present invention which comprises a nucleic acid double strand-stabilizing factor.
Fig. 9 is a schematic diagram of selective amplification results of a deletion mutation using the oligonucleotide fragment of the present invention.

### Detailed description for carrying out the invention

In order to detect a small quantity of a nucleic acid variant to be detected present in a large quantity of nucleic acid variants not to be detected, the inventor has conducted extensive researches and proposed the technical solution of the present invention. In view of the disclosure herein, those skilled in the art can appropriately improve process parameters. It should be particularly indicated that, all similar replacements and changes are obvious for those skilled in the art, which are deemed to be included in the present invention. The applications of the present invention have been disclosed by preferred examples, and apparently, those skilled in the art can make alternations or suitable modifications and combinations to the applications as described herein to achieve and apply the technology of the present invention.

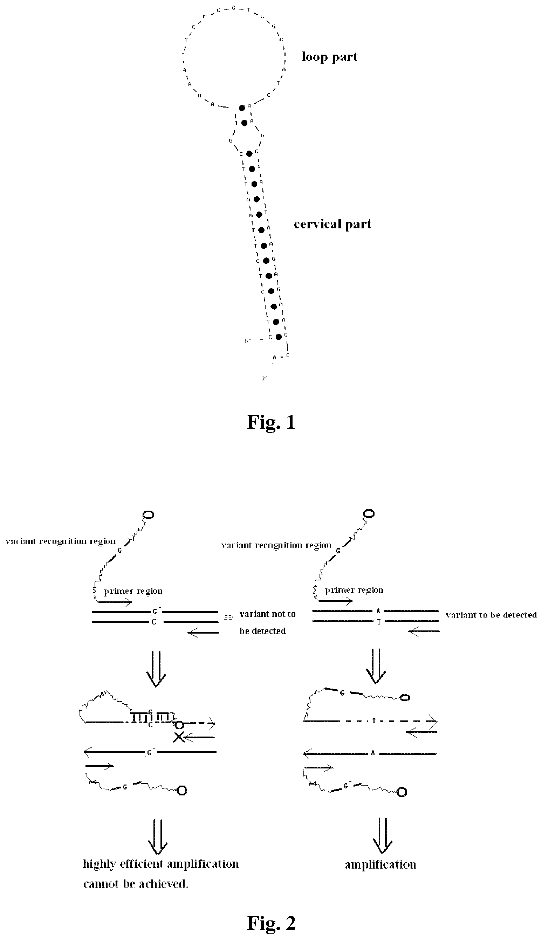

In one aspect, the present invention provides a method for selective amplification of a variant of a target nucleic acid sequence, which comprises amplifying a sample to be detected by using at least an oligonucleotide fragment, wherein the sample may comprises one or more variants. The oligonucleotide fragment comprises a primer region at 3' terminal and a variant recognition region at 5' terminal which are covalently linked. In a suitable reaction system, the primer region is hybridized with the target nucleic acid sequence, and the 3' terminal of the primer region is extended by nucleic acid polymerase. When a variant not to be detected of the target nucleic acid sequence is used as a template, the variant recognition region is completely or partially complementary to a sequence generated from extension of the primer region, forming a hairpin structure, as shown in Fig. 1.

When a nucleic acid variant to be detected is used as a template, the variant recognition region is not completely complementary to a sequence generated from extension of the primer region, failing to form a stable hairpin structure. The hairpin structure as described above is unsuitable as a template for the next round of amplification, inhibits the amplification efficiency and causes the amplification of a variant not to be detected suffering from larger inhibition compared to the variant to be detected, thus achieving selective amplification, as shown in Fig. 2.

In some examples, the variant recognition region is covalently linked with a nucleic acid double strand-stabilizing factor. The double strand-stabilizing factor can increase the annealing temperature of the cervical part of the hairpin structure, serving to stabilize the hairpin structure. In some examples, the variant recognition region comprises a modification which cannot be hydrolyzed by nuclease, including a modification to a base analogue, a modification to a nucleic acid backbone, a modification to a deoxyribose analogue, the modification including but not limited to a peptide nucleic acid, thiophosphate, etc. These modifications can prevent the oligonucleotide fragment from hydrolysis by nuclease, and protect the hairpin structure from being damaged.

In some embodiments, a nucleic acid double strand-stabilizing factor, which is directly linked, or covalently linked via other groups to a variant recognition region, is linked on the nucleotide at the 5' terminal of the oligonucleotide fragment, in which the nucleic acid double strand-stabilizing factor may be a locked nucleic acid, peptide nucleic acid or DNA minor groove binder and analogues thereof, etc.

In some examples, a nucleic acid detection method is used in combination, which includes a nucleic acid detection method which does not distinguish nucleic acid variants, and also includes a detection method which distinguishes different nucleic acid variants. The nucleic acid detection method which does not distinguish nucleic acid variants includes a dye method, hybridization probe detection method, hydrolysis probe detection method, Taqman probe method or molecular beacon method. The detection method which distinguishes different nucleic acid variants includes sequencing or high-resolution melting curve. Alternatively, a method such as a hybridization probe distinguishing different nucleic acid variants, a hydrolysis probe distinguishing different nucleic acid variants, a Taqman probe distinguishing different nucleic acid variants or a molecular beacon distinguishing different nucleic acid variants is used in combination.

In some examples, a suitable reaction system is a system which initiates a reaction with the increase of temperature. Such a system which initiates a reaction with the increase of temperature can increase the specificity of the reaction.

In another aspect, the present invention provides an oligonucleotide fragment consisting of a primer region and a variant recognition region which are covalently linked with each other. When the variant recognition region is completely or partly complementary to a sequence generated from extension of the primer region, a hairpin structure forms. The variant recognition region is covalently linked with a nucleic acid double strand-stabilizing factor or comprises a modification which cannot be hydrolyzed by nuclease, the modification including a modification to a base analogue, a modification to a nucleic acid backbone, a modification to a deoxyribose analogue, the modification including but not limited to a peptide nucleic acid, thiophosphate, etc., wherein the nucleic acid double strand-stabilizing factor may be a locked nucleic acid, peptide nucleic acid or DNA minor groove binder and analogues thereof, etc. The nucleic acid double strand-stabilizing factor, which is directly linked, or covalently linked via other groups to the variant recognition region of the oligonucleotide fragment, is linked on the nucleotide at the 5' terminal of the oligonucleotide fragment.

In another aspect, the present invention provides a kit for detection of a nucleic acid variant, the kit comprising the above-described oligonucleotide fragment, in which the fragment comprises a primer region and a variant recognition region which are covalently linked with each other. When the variant recognition region is completely or partly complementary to a sequence generated from extension of the primer region, a hairpin structure forms. The variant recognition region is covalently linked with a nucleic acid double strand-stabilizing factor or comprises a modification which cannot be hydrolyzed by nuclease, the modification including a modification to a base analogue, a modification to a nucleic acid backbone, a modification to a deoxyribose analogue, the modification including but not limited to a peptide nucleic acid, thiophosphate, etc., wherein the nucleic acid double strand-stabilizing factor may be a locked nucleic acid, peptide nucleic acid or DNA minor groove binder and analogues thereof, etc. The kit further comprises a suitable kit amplification system, such as a system comprising nucleic acid polymerase required for extension, divalent metal ions, nucleotides, salts, buffers and other cofactors, etc. In addition, the kit further comprises a reagent required for nucleic acid detection, which includes but is not limited to one or several of nucleic acid dyes, probes and others.

The oligonucleotide fragment of the present invention comprises at least a primer region and a variant recognition region which may be covalently linked with each other. When the primer region is completely complementary to a target nucleic acid complementary thereto, in a suitable extension system with nucleic acid polymerase, the primer region is extended by the nucleic acid polymerase with nucleotides as raw materials. "Complementary" means that base pairs are formed between the oligonucleotide fragment and its target nucleic acid via hydrogen bonds, including base pairs between adenine A and thymine T or uracil U, base pairs between cytosine C and guanine G, and also including hydrogen bonds formed by participation of some natural or unnatural nucleotide analogues. For example, 5-bromouracil may form a complementary base pair with adenine, similarly with thymine, but in its enol form, it may form a complementary base pair with guanine. The designed oligonucleotide fragments, as well as other primers and probes mentioned in the present invention are shown in Table 1.

**Table 1. Designed oligonucleotide fragments, as well as other primers and probes**

| Primers and probes | |
|---|---|
| oligonucleotide fragments of the present invention | |
| Sequence 4 5' MGB-CTGGG--spacer C3—CACCGTGCCGAACGCA-3' | |
| Sequence 8 5' MGB-GCGTGATGAG--spacer C3—CCTCACCTCCACCGTGCA-3' | |
| Sequence 11 5' MGB-gcccagc-spacer C3-GGAAACTGAATTCAAAAAGATCAAAG-3' | |
| Sequence 15 5' MGB-CTTCTCTTAATTCGTTAAAATTCCCGTCGCTATCAAG-3' | |

| Other primers and probes | |
|---|---|
| Sequence 3 | 5'-TATACACCGTGCCGAACGC-3' |
| Sequence 7 | 5'-AGGCAGCCGAAGGGCAT-3' |
| Sequence 9 | 5'-FAM- GTGCTGaGCTCCGG-3'-QMGB |
| Sequence 10 | 5'-TGGAGCCTCTTACACCCAGTG-3' |
| Sequence 14 | 5'-TTCCTTGTTGGCTTTCGGAG-3' |
| Sequence 16 5'-GCCCAGC-spacer C3-GGAAACTGAATTCAAAAAGATCAAAG-3′ | |

For the above oligonucleotide fragments and other primers and probes, if the modification occurs in the middle of the fragment, they are represented by the modification fragments, such as MGB, spacer C3, FAM or QMGB, together with the two fragments in front of and behind them (see the sequence listing). Sequence 4 is a sequence linked by MGB, sequence 4a, spacer C3 and sequence 4b; Sequence 8 is a sequence linked by MGB, sequence 8a, spacer C3 and sequence 8b; Sequence 11 is a sequence linked by MGB, sequence 11a, spacer C3 and sequence 11b; and Sequence 16 is a sequence linked by sequence 16a, spacer C3 and sequence 16b.

Under suitable conditions, the variant recognition region is completely complementary or not completely complementary to the fragment extended from the primer region. When the variant recognition region is completely or partly complementary to the sequence generated from extension of the primer region, a hairpin structure forms. When the variant recognition region is not completely complementary to the fragment extended from the primer region, there is no stable hairpin structure formed. The above formed hairpin structure is unsuitable as a template for the next round of amplification. In some examples, the variant recognition region is covalently linked with a nucleic acid double strand-stabilizing factor. The double-stranded stabilizing factor can increase the annealing temperature of the cervical part of the hairpin structure, serving to stabilize the hairpin structure. In some examples, the variant recognition region comprises a modification which cannot be hydrolyzed by nuclease, including a modification to a base analogue, a modification to a nucleic acid backbone, a modification to a deoxyribose analogue, the modification including but not limited to a peptide nucleic acid, thiophosphate, etc. These modifications can prevent the oligonucleotide fragment from hydrolysis by nuclease, and protect the hairpin structure from being damaged. The stable hairpin structure inhibits the amplification efficiency, and causes the amplification of wild-type variants to be suffered from larger inhibition as compared to the mutant-type variants, thus achieving the selective amplification, as shown in Fig. 2. In case of the number of the initial nucleic acids being the same, due to the different amplification efficiency, there will also be a very huge difference in number after several cycles, as shown in Table 2.

**Table 2. Difference in number of nucleic acids generated at different amplification efficiency**

| initial number | 100 | 100 | 100 |
|---|---|---|---|
| amplification efficiency | 10% | 50% | 100% |
| number after 10 cycles | 2.59E+02 | 5.77E+03 | 1.02E+05 |
| number after 20 cycles | 6.73E+02 | 3.33E+05 | 1.05E+08 |
| number after 30 cycles | 1.74E+03 | 1.92E+07 | 1.07E+11 |
| number after 40 cycles | 4.53E+03 | 1.11E+09 | 1.10E+14 |

The linkage between the primer region and the variant recognition region, and the linkage between a nucleic acid polymerase extension inhibitor and the oligonucleotide may be a direct covalent bond, such as a 3'-5'phosphodiester bond in a nucleic acid backbone, and also may be other chemical groups including but not limited to alkyl groups, nucleotides and analogues thereof, carbohydrates, nitrogen-containing heterocycles, etc. The length of the linkage between the primer region and the variant recognition region may be zero (i.e., direct linkage), or may be dozens of base pairs in length or other suitable lengths.

Unless particularly defined, all the scientific or technological terminologies in the present invention are the same as general understandings thereof to most of general persons in the art. Most of the terminologies in the art have general meanings in the following documents [Singleton et al., Dictionary of Microbiology and Molecular Biology(2nd ed. 1994)]; [The Cambridge Dictionary of Science and Technology(Walker ed., 1988)]; [The Glossary of Genetics, 5th Ed., R. Rieger et al.(eds.), Springer Verlag(1991)]; [Hale & Marham, The Harper Collins Dictionary of Biology(1991)]. Unless otherwise defined, all professional terms in the present invention are the same as these described in the above documents.

The term "nucleoside" involves a base or basic group covalently linked on a sugar molecule or an analogue thereof. As described above, the base may be a naturally occurring base, and may also be a non-naturally occurring base [Seela et al. (1999) Helv.Chim. Acta 82:1640]. Some bases are for altering the melting temperature (Tm) of a nucleic acid, for example, those including 7-azapurine (such as 7-azaguanine, 7-azaadenine, etc.), or those as mentioned in U.S. Pat. No. 5990303. Other typical heterocyclic bases include but are not limited to: hypoxanthine, inositol, xanthine, derivatives of 2-aminopurine, 2,6-diaminopurine, 2-amino-6-chloropurine, hypoxanthine, inositol, xanthine; as well as 7-azido-8-azo derivatives of adenine, guanine, 2-aminopurine, 2,6-diaminopurine, 2-amino-6-chloropurine, hypoxanthine, inositol, xanthine; 6-azacytidine; 5-fluorocytosine; 5-chlorocytosine; 5-iodocytosine; 5-bromocytosine; 5-methylcytosine; 5-propynylcytosine; 5-bromovinyluracil; 5-fluorouracil; 5-chlorouracil; 5-iodouracil; 5-bromouracil; 5-trifluoromethyluracil; 5-methoxyuracil; 5-ethynyluracil; 5-propynyluracil; etc. Typical nucleosides include but are not limited to ribonucleoside, deoxyribonucleoside, dideoxyribonucleoside and carbocyclic nucleoside.

The term "nucleotide" generally refers to a compound which is formed from a nucleoside linked to an acidic molecule or group via an ester bond, for example, nucleoside phosphate, which commonly has one, two or three phosphate groups covalently linked on position 5 of the glycosyl group in the nucleoside. In some cases, the definition of the nucleotide also involves homologues or analogues of some typical nucleotides.

The term "oligonucleotide" refers to a multimer which is formed from nucleotides which are linked therebetween via a covalent bond. An oligonucleotide generally comprises at least 3 nucleotides. In some cases, an oligonucleotide possibly comprises linkages of phosphamide [Beaucage et al. (1993) Tetrahedron 49(10):1925], thiophosphate [Mag et al.(1991) Nucleic Acids Res. 19:1437; and U.S. Patent No. 5644048], dithiophophate [Briu et al. (1989) J. Am. Chem. Soc. 111:2321], O-methyl phosphamide [Eckstein, Oligonucleotides and Analogues: A Practical Approach, Oxford University Press(1992)], or a peptide nucleic acid backbone linkage [Egholm (1992) J. Am. Chem. Soc. 114:1895]. Other oligonucleotides further includes those positively charged backbones [Denpcy et al. (1995) Proc. Natl. Acad. Sci. USA 92: 6097], non-ionic type backbones (U.S. Pat. Nos. 5386023, 5637684, 5602240, 5216141 and 4469863) and non-ribose backbones (U.S. Pat. Nos. 5235033 and 5034506). An oligonucleotide comprising one or more carbocyclic sugars also falls within the definition of the nucleic acid [Jenkins et al.(1995) Chem. Soc. Rev. pp. 169-176]. Those situations in which a modification is performed on a ribose-phosphate backbone for a purpose of improving stability of a molecule under a specific condition or tagging an oligonucleotide are also encompassed in the definition scope of the oligonucleotide. The oligonucleotide may also comprises various spacer modifications, such as Spacer C3, Spacer C9, Spacer C18, etc.

The term "nucleic acid" includes deoxyribonucleic acids (DNAs), ribonucleic acids (RNAs), DNA-RNA hybrids, oligonucleotides, aptamers, peptide nucleic acids (PNAs), PNA-DNA hybrids, PNA-RNA hybrids, and the like. All covalently linked nucleotides in a linear (single-stranded or double-stranded) or branched form are comprised. A typical nucleic acid is generally single-stranded or double-stranded, and comprises a phosphodiester bond.

The term "hairpin structure" refers to a structure which is formed from hybridization of a sequence in a nucleic acid with another sequence of itself. A typical hairpin structure is shown in Fig. 1.

The term "target nucleic acid sequence" refers to a nucleic acid sequence to be amplified, which is used as a template for nucleic acid amplification.

The term "variant of a nucleic acid sequence", "nucleic acid variant" refer to a specific nucleic acid sequence with difference between different variants, in which the difference may be difference in a single or more bases, and may be an insertion, deletion, translocation or a combination of all the above types.

The term "wild-type variant" refers to a "normal" gene sequence which is naturally occurring, and it is a sequence present at the highest frequency in a same gene sequence in a majority of populations.

The term "mutant-type variant" refers to a nucleic acid sequence different from the "wild-type variant". Such a difference may be difference in a single or more bases, and may be an insertion or deletion or a combination of all the above types, for example, a mutated nucleic acid sequence in tumor cells. In some examples, compared to the wild-type variant, the mutant-type variant accounts for very low percentage.

The term "primer region" refers to an oligonucleotide sequence complementary to one strand of a target nucleic acid sequence, which is generally used as a starting site for extension by nucleic acid polymerase.

The term "variant recognition region" refers to an oligonucleotide sequence which is often paired to a sequence having difference from a nucleic acid variant, and different nucleic acid variants can be distinguished depending on various degrees of pairing.

The term "nucleic acid polymerase" refers to an enzyme which is capable of incorporating nucleotides into a nucleic acid through a biocatalytic reaction, including but not limited to DNA polymerase, RNA polymerase, reverse transcriptase, terminal transferase, etc.

The term "extension" refers to a process that an oligonucleotide fragment is covalently linked to newly incorporated nucleotides by using nucleic acid polymerase, to form a new oligonucleotide.

The term "amplification" refers to a process that the number of a target nucleic acid fragment is increased under the action of nucleic acid polymerase, which includes but is not limited to polymerase chain reaction (PCR), ligase chain reaction (LCR), nucleic acid sequence-based amplification (NASBA), transcription-mediated amplification (TMA), loop-mediated isothermal amplification (LAMP), strand displacement amplification (SDA), helicase-dependent amplification (HDA), etc.

In examples of the present invention, amplification refers to a polymerase chain reaction (PCR). After denaturation and melting of a template, an oligonucleotide primer is annealed and hybridized with the template, accompanied by extension by addition of nucleotides, and through certain rounds of such repeated cycles, amplification of the target nucleotide fragment is achieved. Sometimes, annealing and extension are performed at the same temperature.

The term "completely complementary", when used for the description of an oligonucleotide chain in the present invention, refers to that each base of the oligonucleotide chain forms a Waston-Crick base pair or a similar hydrogen bond pair with another oligonucleotide chain in the double-stranded nucleic acid or a base in the nucleic acid chain. "Partly complementary", when used for the description of an oligonucleotide chain in the present invention, refers to that more than one but not all bases in the oligonucleotide chain form Waston-Crick base pairs or similar hydrogen bond pairs with another oligonucleotide chain or a base in the nucleic acid.

The term "mismatched" nucleic acid refers to a nucleic acid comprising one or more than one mismatched base pair(s). The mismatched base pair refers to that two corresponding bases in a double-stranded nucleic acid cannot form a Waston-Crick base pair or a similar hydrogen bond pairs.

The term "nucleic acid double strand-stabilizing factor" in the present invention refers to a compound or group which can facilitate to stabilize the double-stranded structure of a nucleic acid. It may be a locked nucleic acid, and also may be a peptide nucleic acid or DNA minor groove binder. Preferably, the modification is a DNA minor groove binder. It has been previously reported in literatures that a DNA minor groove binder molecule can significantly increase the annealing temperature of a hybrid nucleic acid dimer, and commonly, the DNA minor groove binder links to 3' terminal of an oligonucleotide fragment. Because the linked DNA minor groove binder can significantly increase the annealing temperature of a hybrid nucleic acid dimer, different complementarity between different nucleic acid variants and the modified oligonucleotide fragment produces large difference in the annealing temperature, thus producing different inhibition effects to extension by nucleic acid polymerase. In the present invention, preferably, the DNA minor groove binder connects to 5' terminal of the oligonucleotide.

The term "minor groove binder" refers to a compound which is capable of binding to a DNA minor groove. A DNA double helix has two grooves on its surface, named a major groove and minor groove, respectively. The DNA major groove possesses more information about hydrogen bond donors, acceptors, charges, etc., as compared to the DNA minor groove, and intracellular proteins prefer to recognize sites within the major groove to control gene expressions or exert other intracellular functions.

The DNA minor groove is a target for some antibiotics in nature, such as netropsin, distamycin. Both the compounds have a dissociation constant of 10⁻⁵ M orders of magnitude when binding DNA, and exhibit a preference to a DNA AT-rich region. Both the two compounds have some limitations on aspects such as selectivity, toxicity, etc. A lot of similar compounds thus have been found by the researches to overcome these limitations, for example, compounds mentioned in the following reviews: [Sondhi et al. (Curr. Med. Chem. 4, 313 (1997)), [Reddy et al. (Pharmacology & Therapeutics 84, 1(1999)], Wemmer (Biopolymers 52, 197(2001)], [Dervan (Bioorg. Med. Chem. 9, 2215(2001)].

It is said that there are also some compounds which prefer to bind GC base pair region in DNA, for example, compounds mentioned in the following documents: [Anti-Cancer Drug Design 5,3 (1990)], [ Proc. Natl. Acad. Sci. USA 89,7586 (1992)], [Biochemistry 32,4237 (1993)], [Science 266,647 (1994)], [Anti-Cancer Drug Design 10,155 (1995) ], [Bioorg Med. Chem. 8, 985 (2000)], [Mol. Biol. 34,357 (2000)]. Different analogues of netropsin and distamycin have been found, for example, [J Am. Chem. Soc.114 (15), 5911 (1992)], [Biochemistry 31, 8349 (1992) ], [Bioconjugate Chem.5,475 (1994) ], [ Biochem. Biophys. Res. Commun. 222,764 (1996)], [J Med.Chem. 43,3257 (2000)], [Tetrahedron 56,5225 (2000) ], [Molecular Pharmacology 54,280 (1998)], [Bioorg Med. Chem. Lett. 6 (18), 2169 (1996) ], [J. Med. Chem. 45, 805 (2002)], [Bioorg. Med. Chem. Lett. 12,2007 (2002)], (international patent applications WO 97/28123, WO 98/21202, WO 01/74898 and WO 02/00650, US patent numbers 4,912,199, 5,273,991, 5,637,621, 5,698,674 and 5,753,629). WO/2003/059881 further discloses polypeptide homologues of netropsin and distamycin.

The term "sequencing" refers to analysis of the sequence of base for a specific nucleic acid fragment, that is, the arrangement of adenine (A), thymine (T), cytosine (C) and guanine (G). Sanger method is a method which starts from a certain fixed site based on a nucleotide and randomly terminates at a specific base, and makes florescence labeling after each base, to produce four groups of a series of nucleotides of different length which are ended with adenine (A), thymine (T), cytosine (C) and guanine (G), followed by detection via electrophoresis on urea-denatured PAGE gels, thus obtaining visible sequences of DNA bases. In cyclic array sequencing by synthesis, all the sequences are indirectly determined by reading optical signals released in a process of linking a base onto a DNA chain with DNA polymerase or DNA ligase, by the aid of fluorescence or a chemiluminescent material. Novel nanopore sequencing is sequencing method using an electrophoretic technique which drives a single molecule through the nanopore one by one by means of electrophoresis. Due to the very small diameter of the nanopore, it allows only a single nucleic acid polymer to pass through, and different bases have different charging properties, which makes it possible to detect the type of the passed base by difference in electrical signals, thus achieving sequencing. There are a variety of methods for nucleic acid sequencing, and new methods are in continuous development.

The term "dye method" refers to a nucleic acid detection method which obtains signals by using a dye that interacts with the nucleic acid.

The term "high-resolution melting curve method" refers to real-time detection of changes of fluorescent signals in melting processes of different nucleic acids with a fluorescent dye incorporated in a nucleic acid, allowing intuitive display of difference present in different nucleic acids by way of generation of melting curves in different profiles.

The term "hybridization probe detection method" refers to a method to detect a nucleic acid by forming complementary base pairings between a small section of a nucleic acid with a possibly detected signal molecule and a nucleic acid to be detected to generate signal changes. The probe may be a probe distinguishing different nucleic acid variants, and may also be a probe not distinguishing different nucleic acid variants.

The term "hydrolysis probe detection method" refers to a method that in presence of a nucleic acid to be detected, a small section of a nucleic acid with a possibly detected signal molecule is hydrolyzed by nuclease to produce a signal change for detecting the nucleic acid. The probe may be a probe distinguishing different nucleic acid variants, and may also be a probe not distinguishing different nucleic acid variants.

The term "Taqman probe method" is a Taqman^{™} probe method as disclosed in U.S. Patent (U.S. Pat. Nos. 5,210,015, 5,538,848 and 6,326,145), in which by specific hybridization of a probe to a nucleic acid fragment to be detected, and by cleavage with DNA polymerase having 5' exonuclease activity, the generated signal is used to indicate the presence of a target fragment to be detected. The probe may be a probe distinguishing different nucleic acid variants, and may be a probe not distinguishing different nucleic acid variants.

The term "molecular beacon method" refers to a method to detect a nucleic acid to be detected by a molecular beacon. The molecular beacon is a double-labeling cervical-loop oligonucleotide probe having a hairpin structure of about 8 bases formed by itself at its 5' and 3' terminal, the nucleic acid sequences at the two terminals being complementary to each other, and thus, a fluorescent group labeled at one terminal comes closely proximate to a quenching group labeled at another terminal, thus generating no fluorescence. In the presence of a nucleic acid to be detected, the molecular beacon is hybridized to the nucleic acid to be detected, thus making the fluorescent group far away from the quenching group to produce an enhanced fluorescent signal for detecting the nucleic acid to be detected. The molecular beacon may be an oligonucleotide probe distinguishing different nucleic acid variants, and may also be an oligonucleotide probe not distinguishing different nucleic acid variants.

In examples of the present invention, an amplification reaction comprises a process of hot start. The selectivity of the selective amplification in the present invention may be enhanced due to the hot start. There are a variety of hot start methods in the art, for example, the use of wax to separate key components from remaining components in a reaction, and with the temperature increasing, the wax melting making the key components meet the remaining components, thus allowing the reaction to occur (U.S. Pat. No. 5411876); the use of an antibody to allow reversible inactivation of nucleic acid polymerase (US Pat 5338671), or the use of an oligonucleotide which binds to nucleic acid polymerase to inactivate the nucleic acid polymerase (US Pat 5840867). Alternatively, nucleic acid polymerase with reversible chemical modifications is used to achieve the effect of the hot start (US Pat 5677152, Pat 5773528).

In examples of the present invention, a nucleic acid amplification test is a fluorescent quantitative PCR test. In a fluorescent quantitative PCR reaction, a parameter to measure the amplification is Ct value, an earlier Ct value indicates that the signal reaches a threshold value more rapidly. Ct difference between different nucleic acid variants in an amplification sample usually reflects difference of the amplification efficiency of an amplification system for different nucleic acid variants, which further reflects the selectivity of the amplification system.

There are also a variety of methods for detection of an amplified product in the art. These methods include the use of a fluorescent labeled probe, or various dyes binding to a nucleic acid. Such detection may be specific detection of one or more of nucleic acid variants, and may also be nonspecific detection of all the signals of nucleic acids. The detection of an amplified product may be performed after completion of the amplification reaction, for example, by a method of gel electrophoresis, or a method of staining a nucleic acid. In addition, the detection of an amplified product may be performed during the amplification reaction. The selectivity of the method of the present invention may be further enhanced by the method for selective amplification of the present invention in combination with a method for selective detection distinguishing different nucleic acid variants.

### EXAMPLE 1: Selective amplification of EGFR G719S using an oligonucleotide fragment provided in the present invention

In this example, two variants of a target nucleic acid sequence were added in an amount of 104 copies and 105 copies respectively, in which one variant was a plasmid DNA which had been inserted with a EGFR G719S mutant-type sequence (SEQ ID NO: 1), while the other variant was the same plasmid which had been inserted with a EGFR wild-type sequence (SEQ ID NO: 2).
(EGFR G719S mutant-type sequence fragment): SEQ ID NO: 1 
TTTCCAGCATGGTGAGGGCTGAGGTGACCCTTGTCTCTGTGTTCTTG
TCCCCCCCAGCTTGTGGAGCCTCTTACACCCAGTGGAGAAGCTCCCAACC
AAGCTCTCTTGAGGATCTTGAAGGAAACTGAATTCAAAAAGATCAAAGTG
CTGaGCTCCGGTGCGTTCGGCACGGTGTATAAGGTAAGGTCCCTGGCACAG
GCCTCTGGGCTGGGCCGCAGGGCCTCTCATGGTCTGGTGGGGAGCCCAGA
GTCCTTGCAAGCTGTATATTTCCATCATCTACTTTACTCTTTGTTTCACTGAG
 T
(EGFR G719S wild-type sequence fragment): SEQ ID NO: 2

TTTCCAGCATGGTGAGGGCTGAGGTGACCCTTGTCTCTGTGTTCTTG
TCCCCCCCAGCTTGTGGAGCCTCTTACACCCAGTGGAGAAGCTCCCAACC
AAGCTCTCTTGAGGATCTTGAAGGAAACTGAATTCAAAAAGATCAAAGTG
CTGGGCTCCGGTGCGTTCGGCACGGTGTATAAGGTAAGGTCCCTGGCACA
GGCCTCTGGGCTGGGCCGCAGGGCCTCTCATGGTCTGGTGGGGAGCCCAG
AGTCCTTGCAAGCTGTATATTTCCATCATCTACTTTACTCTTTGTTTCACTGA
GT

The sequence of a forward primer was Sequence 10, and an oligonucleotide fragment provided in the present invention was used as a reverse primer, of which the sequence was Sequence 4 (see Table 1).

PCR reaction systems had a volume of 25 µl, with each reaction system comprising 2% glycerol, and dATP, dCTP, dGTP, dTTP each at a concentration of 200 µM. The forward primer (Sequence 10) and the oligonucleotide fragment provided in the present invention as the reverse primer (Sequence 4) both had a concentration of 200 nM, and SYBR Green had a concentration of 0.4*, with 5 units of hot-start Taq DNA polymerase.

A Roche LightCycle 480 fluorescent quantitative PCR instrument was used for PCR reactions and subsequent data analysis. Thermal cycle conditions for PCR were as follows: 10 min at 95°C; 45 cycles (15 s at 95°C, 40 s at 60°C, single); melting (1 min at 95°C, 2 min at 40°C, continuous at 99°C); cool (30 s at 37°C).

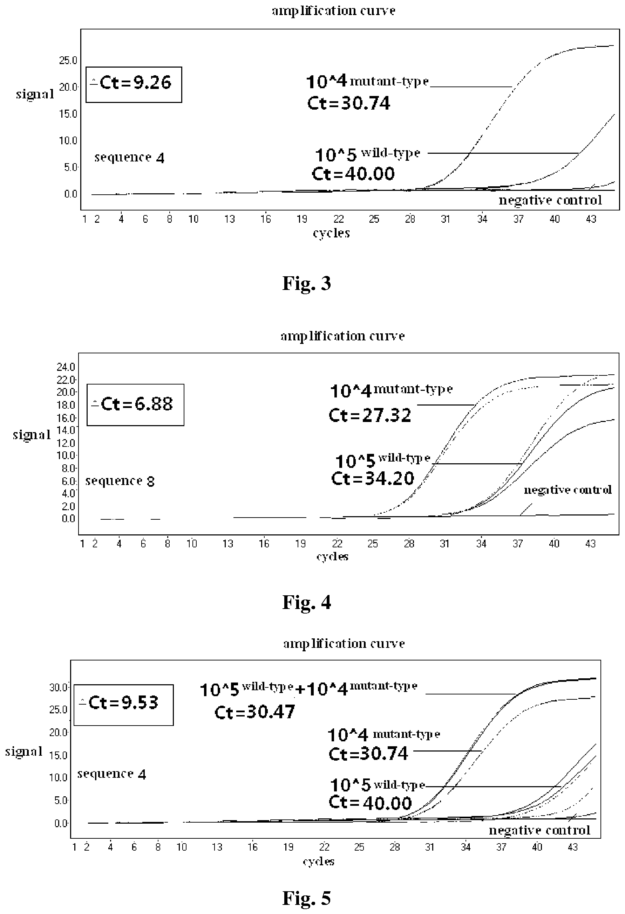

The experimental results are shown in Fig. 3, in which the experimental results are embodied by fluorescence changes at 465 nM-510 nM, while the selectivity of the system is embodied by difference in Ct values for amplification of different variant templates, with delta Ct values indicated in Fig. 3. These experimental results show that selective amplification of EGFR G719S was achieved by amplification of different variant templates using the oligonucleotide fragment provided in the present invention.

### EXAMPLE 2: Selective amplification of EGFR T790M using an oligonucleotide fragment provided in the present invention

In this example, two variants of a target nucleic acid sequence were added in an amount of 104 copies and 105 copies respectively, in which one variant was a plasmid DNA which had been inserted with a EGFR T790M mutant-type sequence (SEQ ID NO: 5), while the other variant was the same plasmid which had been inserted with a EGFR wild-type sequence (SEQ ID NO: 6).
(EGFR T790M mutant-type sequence fragment): SEQ ID NO: 5 
CTGGCCACCATGCGAAGCCACACTGACGTGCCTCTCCCTCCCTCCAG
GAAGCCTACGTGATGGCCAGCGTGGACAACCCCCACGTGTGCCGCCTGCT
GGGCATCTGCCTCACCTCCACCGTGCAGCTCATCATGCAGCTCATGCCCTT
CGGCTGCCTCCTGGACTATGTCCGGGAACACAAAGACAATATTGGCTCCCA
GTACCTGCTCAACTGGTGTGTGCAGATCGCAAAGGTAATCAGGGAAGGGA
GATACGGGGAGGGGAGATAAGGAGCCAGGATCCT
(EGFR T790M wild-type sequence fragment): SEQ ID NO: 6

CTGGCCACCATGCGAAGCCACACTGACGTGCCTCTCCCTCCCTCCAG
GAAGCCTACGTGATGGCCAGCGTGGACAACCCCCACGTGTGCCGCCTGCT
GGGCATCTGCCTCACCTCCACCGTGCAGCTCATCACGCAGCTCATGCCCTT
CGGCTGCCTCCTGGACTATGTCCGGGAACACAAAGACAATATTGGCTCCCA
GTACCTGCTCAACTGGTGTGTGCAGATCGCAAAGGTAATCAGGGAAGGGA
GATACGGGGAGGGGAGATAAGGAGCCAGGATCCT

The sequence of a forward primer was Sequence 7, and an oligonucleotide fragment provided in the present invention was used as a reverse primer, of which the sequence was Sequence 8 (see Table 1).

PCR reaction systems had a volume of 25 µl, with each reaction system comprising 2% glycerol, and dATP, dCTP, dGTP, dTTP each at a concentration of 200 µM. The forward primer (Sequence 7) and the oligonucleotide fragment provided in the present invention as the reverse primer (Sequence 8) both had a concentration of 200 nM, and SYBR Green had a concentration of 0.4*, with 5 units of hot-start Taq DNA polymerase.

A Roche LightCycle 480 fluorescent quantitative PCR instrument was used for PCR reactions and subsequent data analysis. Thermal cycle conditions for PCR were as follows: 10 min at 95°C; 45 cycles (15 s at 95°C, 40 s at 60°C, single); melting (1 min at 95°C, 2 min at 40°C, continues at 99°C); cool (30 s at 37°C).

The experimental results are shown in Fig. 4, in which the experimental results are embodied by fluorescence changes at 465 nM-510 nM, while the selectivity of the system is embodied by difference in Ct values for amplification of different variant templates, with delta Ct values indicated in Fig. 4. These experimental results show that selective amplification of EGFR T790M was achieved using the oligonucleotide fragment provided in the present invention.

### EXAMPLE 3: Selective amplification of a mixed template using an oligonucleotide fragment provided in the present invention

In this example, two variants of a target nucleic acid sequence were added in an amount of 104 copies and 105 copies respectively, in which one variant was a plasmid DNA which had been inserted with a EGFR G719S mutant-type sequence (SEQ ID NO: 1), while the other variant was the same plasmid which had been inserted with a EGFR wild-type sequence (SEQ ID NO: 2).
(EGFR G719S mutant-type sequence fragment): SEQ ID NO: 1 
TTTCCAGCATGGTGAGGGCTGAGGTGACCCTTGTCTCTGTGTTCTTG
TCCCCCCCAGCTTGTGGAGCCTCTTACACCCAGTGGAGAAGCTCCCAACC
AAGCTCTCTTGAGGATCTTGAAGGAAACTGAATTCAAAAAGATCAAAGTG
CTGaGCTCCGGTGCGTTCGGCACGGTGTATAAGGTAAGGTCCCTGGCACAG
GCCTCTGGGCTGGGCCGCAGGGCCTCTCATGGTCTGGTGGGGAGCCCAGA
GTCCTTGCAAGCTGTATATTTCCATCATCTACTTTACTCTTTGTTTCACTGAG
T
(EGFR G719S wild-type sequence fragment): SEQ ID NO: 2

TTTCCAGCATGGTGAGGGCTGAGGTGACCCTTGTCTCTGTGTTCTTG
TCCCCCCCAGCTTGTGGAGCCTCTTACACCCAGTGGAGAAGCTCCCAACC
AAGCTCTCTTGAGGATCTTGAAGGAAACTGAATTCAAAAAGATCAAAGTG
CTGGGCTCCGGTGCGTTCGGCACGGTGTATAAGGTAAGGTCCCTGGCACA
GGCCTCTGGGCTGGGCCGCAGGGCCTCTCATGGTCTGGTGGGGAGCCCAG
AGTCCTTGCAAGCTGTATATTTCCATCATCTACTTTACTCTTTGTTTCACTGA
GT

The sequence of a forward primer was Sequence 10, and an oligonucleotide fragment provided in the present invention was used as a reverse primer, of which the sequence was Sequence 4 (see Table 1).

PCR reaction systems had a volume of 25 µl, with each reaction system comprising 2% glycerol, and dATP, dCTP, dGTP, dTTP each at a concentration of 200 µM. The forward primer (Sequence 10) and the oligonucleotide fragment provided in the present invention as the reverse primer (Sequence 4) both had a concentration of 200 nM, and SYBR Green had a concentration of 0.4*, with 5 units of hot-start Taq DNA polymerase.

A Roche LightCycle 480 fluorescent quantitative PCR instrument was used for PCR reactions and subsequent data analysis. Thermal cycle conditions for PCR were as follows: 10 min at 95°C; 45 cycles (15 s at 95°C, 40 s at 60°C, single); melting (1 min at 95°C, 2 min at 40°C, continues at 99°C); cool (30 s at 37°C).

The experimental results are shown in Fig. 5, in which the experimental results are embodied by fluorescence changes at 465 nM-510 nM, while the selectivity of the system is embodied by difference in Ct values for amplification of different variant templates, with delta Ct values indicated in Fig. 5. These experimental results show that selective amplification of a mixed template was achieved using the oligonucleotide fragment provided in the present invention.

### EXAMPLE 4: Selective amplification and selective detection using an oligonucleotide fragment provided in the present invention in combination with a MGB probe

In this example, two variants of a target nucleic acid sequence were added in an amount of 100 copies and 104 copies respectively, in which one variant was a plasmid DNA which had been inserted with a EGFR G719S mutant-type sequence (SEQ ID NO: 1), while the other variant was the same plasmid which had been inserted with a EGFR wild-type sequence (SEQ ID NO: 2).
(EGFR G719S mutant-type sequence fragment): SEQ ID NO: 1 
TTTCCAGCATGGTGAGGGCTGAGGTGACCCTTGTCTCTGTGTTCTTG
TCCCCCCCAGCTTGTGGAGCCTCTTACACCCAGTGGAGAAGCTCCCAACC
AAGCTCTCTTGAGGATCTTGAAGGAAACTGAATTCAAAAAGATCAAAGTG
CTGaGCTCCGGTGCGTTCGGCACGGTGTATAAGGTAAGGTCCCTGGCACAG
GCCTCTGGGCTGGGCCGCAGGGCCTCTCATGGTCTGGTGGGGAGCCCAGA
GTCCTTGCAAGCTGTATATTTCCATCATCTACTTTACTCTTTGTTTCACTGAG
T
(EGFR G719S wild-type sequence fragment): SEQ ID NO: 2

TTTCCAGCATGGTGAGGGCTGAGGTGACCCTTGTCTCTGTGTTCTTG
TCCCCCCCAGCTTGTGGAGCCTCTTACACCCAGTGGAGAAGCTCCCAACC
AAGCTCTCTTGAGGATCTTGAAGGAAACTGAATTCAAAAAGATCAAAGTG
CTGGGCTCCGGTGCGTTCGGCACGGTGTATAAGGTAAGGTCCCTGGCACA

GGCCTCTGGGCTGGGCCGCAGGGCCTCTCATGGTCTGGTGGGGAGCCCAG
AGTCCTTGCAAGCTGTATATTTCCATCATCTACTTTACTCTTTGTTTCACTGA
GT

The sequence of a forward primer was Sequence 10, and an oligonucleotide fragment provided in the present invention was used as a reverse primer, of which the sequence was Sequence 4. The sequence of a MGB probe was SEQ ID NO: 9 (see Table 1).

PCR reaction systems had a volume of 25 µl, with each reaction system comprising 2% glycerol, and dATP, dCTP, dGTP, dTTP each at a concentration of 200 µM. The forward primer (Sequence 10) and the oligonucleotide fragment provided in the present invention as the reverse primer (Sequence 4) and probe (Sequence 9) all had a concentration of 200 nM, with 1 unit of hot-start Taq DNA polymerase.

A Roche LightCycle 480 fluorescent quantitative PCR instrument was used for PCR reactions and subsequent data analysis. Thermal cycle conditions for PCR were as follows: 10 min at 95 °C; 45 cycles (15 s at 95°C, 40 s at 60°C, single).

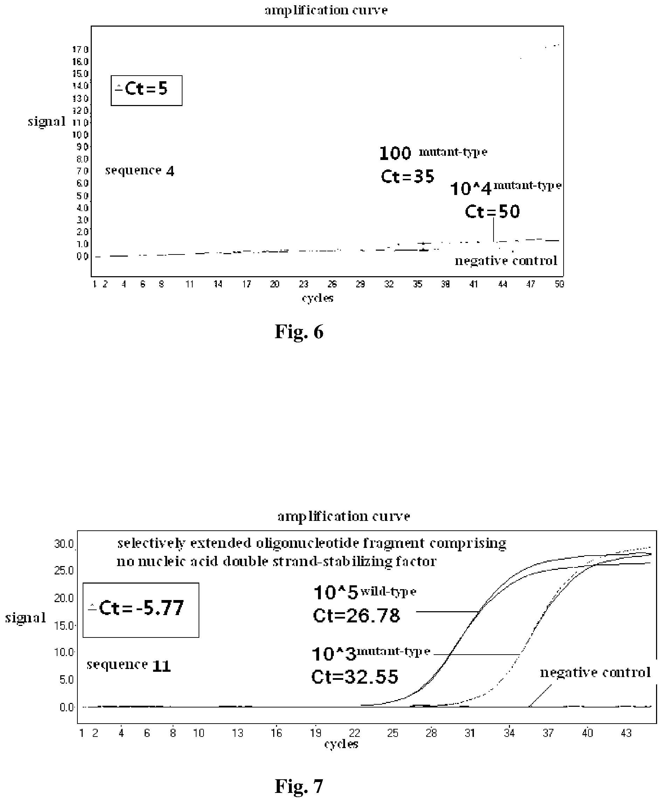

The experimental results are shown in Fig. 6, in which the experimental results are embodied by fluorescence changes at 465 nM-510 nM, while the selectivity of the system is embodied by difference in Ct values for amplification of different variant templates, with delta Ct values indicated in Fig. 6. These experimental results show that selective amplification and selective detection were achieved using the oligonucleotide fragment provided in the present invention in combination with a MGB probe.

### EXAMPLE 5: Comparison between an oligonucleotide fragment comprising a nucleic acid double strand-stabilizing factor mentioned in the present invention and an oligonucleotide fragment comprising no nucleic acid double strand-stabilizing factor mentioned in the present invention

In this example, two variants of a target nucleic acid sequence were of the same amount, in which one variant was a plasmid DNA which had been inserted with a EGFR G719S mutant-type sequence (SEQ ID NO: 1), while the other variant was the same plasmid which had been inserted with a EGFR wild-type sequence (SEQ ID NO: 2).
(EGFR G719S mutant-type sequence fragment): SEQ ID NO: 1 TTTCCAGCATGGTGAGGGCTGAGGTGACCCTTGTCTCTGTGTTCTTG 
TCCCCCCCAGCTTGTGGAGCCTCTTACACCCAGTGGAGAAGCTCCCAACC
AAGCTCTCTTGAGGATCTTGAAGGAAACTGAATTCAAAAAGATCAAAGTG
CTGaGCTCCGGTGCGTTCGGCACGGTGTATAAGGTAAGGTCCCTGGCACAG
GCCTCTGGGCTGGGCCGCAGGGCCTCTCATGGTCTGGTGGGGAGCCCAGA
GTCCTTGCAAGCTGTATATTTCCATCATCTACTTTACTCTTTGTTTCACTGAG
T
(EGFR G719S wild-type sequence fragment): SEQ ID NO: 2

TTTCCAGCATGGTGAGGGCTGAGGTGACCCTTGTCTCTGTGTTCTTG
TCCCCCCCAGCTTGTGGAGCCTCTTACACCCAGTGGAGAAGCTCCCAACC
AAGCTCTCTTGAGGATCTTGAAGGAAACTGAATTCAAAAAGATCAAAGTG
CTGGGCTCCGGTGCGTTCGGCACGGTGTATAAGGTAAGGTCCCTGGCACA
GGCCTCTGGGCTGGGCCGCAGGGCCTCTCATGGTCTGGTGGGGAGCCCAG
AGTCCTTGCAAGCTGTATATTTCCATCATCTACTTTACTCTTTGTTTCACTGA
GT

The sequence of a forward primer was Sequence 3. In some experiment groups, the oligonucleotide as the reverse primer had been modified with a double-stranded stabilizing factor at 5' terminal, of which the sequence was Sequence 11; while in some experimental groups, the oligonucleotide as the reverse primer had not been modified with a double-stranded stabilizing factor, of which the sequence was Sequence 16 (see Table 1).

PCR reaction systems had a volume of 25 µl, with each reaction system comprising 2% glycerol, and dATP, dCTP, dGTP, dTTP each at a concentration of 200 µM. The forward primer (Sequence 3) and the oligonucleotide fragment provided in the present invention as the reverse primer (Sequence 11 and Sequence 16) both had a concentration of 200 nM, and SYBR Green had a concentration of 0.4*, with 5 units of hot-start Taq DNA polymerase.

A Roche LightCycle 480 fluorescent quantitative PCR instrument was used for PCR reactions and subsequent data analysis. Thermal cycle conditions for PCR were as follows: 10 min at 95°C; 45 cycles (15 s at 95°C, 40 s at 60°C, single); melting (1 min at 95°C, 2 min at 40°C, continues at 99°C); cool (30 s at 37°C).

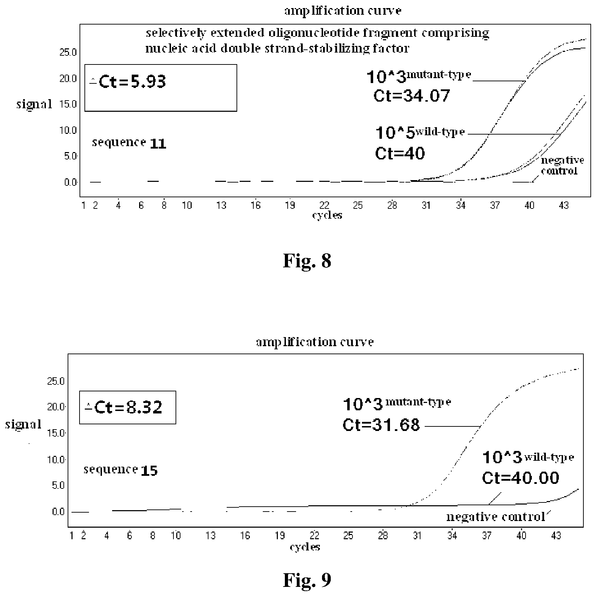

The experimental results are shown in Figure 7 and Figure 8, in which the experimental results are embodied by fluorescence changes at 465 nM-510 nM, while the selectivity of the system is embodied by difference in Ct values for amplification of different variant templates, with delta Ct values indicated in Figure 7 and Figure 8. These experimental results show that the oligonucleotide fragment of the present invention comprising a nucleic acid double strand-stabilizing factor is more selective than the oligonucleotide fragment of the present invention comprising no nucleic acid double strand-stabilizing factor.

### EXAMPLE 6: Selective amplification of a deletion mutation using an oligonucleotide fragment provided in the present invention

In this example, two variants of a target nucleic acid sequence were of the same amount, in which one variant was a plasmid DNA which had been inserted with a EGFR mutant-type sequence with an exon 19 deletion (SEQ ID NO: 12), while the other variant was the same plasmid which had been inserted with a EGFR wild-type sequence with exon 19 (SEQ ID NO: 13).
EGFR 19 extron-deleted mutant-type sequence (SEQ ID NO: 12) 
TTCGGGGTGCATCGCTGGTAACATCCACCCAGATCACTGGGCAGCAT
GTGGCACCATCTCACAATTGCCAGTTAACGTCTTCCTTCTCTCTCTGTCATA
GGGACTCTGGATCCCAGAAGGTGAGAAAGTTAAAATTCCCGTCGCTATCAA
GACATCTCCGAAAGCCAACAAGGAAATCCTCGATGTGAGTTTCTGCTTTGC
TGTGTGGGGGTCCATGGCTCTGAACCTCAGGCCCACCTTTTCTCATGTCTG
GCAGCTGCTCTGCTCTAGACCCTGCTCATCTCCACATCCTAAATGTTCACTT
TC
EGFR 19 extron wild-type sequence (SEQ ID NO: 13)

TTCGGGGTGCATCGCTGGTAACATCCACCCAGATCACTGGGCAGCAT
GTGGCACCATCTCACAATTGCCAGTTAACGTCTTCCTTCTCTCTCTGTCATA
GGGACTCTGGATCCCAGAAGGTGAGAAAGTTAAAATTCCCGTCGCTATCAA
GGAATTAAGAGAAGCAACATCTCCGAAAGCCAACAAGGAAATCCTCGATG
TGAGTTTCTGCTTTGCTGTGTGGGGGTCCATGGCTCTGAACCTCAGGCCCA
CCTTTTCTCATGTCTGGCAGCTGCTCTGCTCTAGACCCTGCTCATCTCCACA
TCCTAAATGTTCACTTTC

The sequence of a forward primer was Sequence 14, and an oligonucleotide fragment provided in the present invention was used as a reverse primer, of which the sequence was Sequence 15.

PCR reaction systems had a volume of 25 µl, with each reaction system comprising 2% glycerol, and dATP, dCTP, dGTP, dTTP each at a concentration of 200 µM. The forward primer (Sequence 14) and the oligonucleotide fragment provided in the present invention as the reverse primer (Sequence 15) both had a concentration of 200 nM, and SYBR Green had a concentration of 0.4*, with 5 units of hot-start Taq DNA polymerase.

A Roche LightCycle 480 fluorescent quantitative PCR instrument was used for PCR reactions and subsequent data analysis. Thermal cycle conditions for PCR were as follows: 10 min at 95°C; 45 cycles (15 s at 95°C, 40 s at 60°C, single); melting (1 min at 95°C, 2 min at 40°C, continues at 99°C); cool (30 s at 37°C).

The experimental results are shown in Fig. 9, in which the experimental results are embodied by fluorescence changes at 465 nM-510 nM, while the selectivity of the system is embodied by difference in Ct values for amplification of different variant templates, with delta Ct values indicated in Fig. 9. These experimental results show that selective amplification of a deletion mutation was achieved using the oligonucleotide fragment provided in the present invention.

The structures of the above-mentioned modification fragments Spacer C3, MGB, Q-MGB and FAM are as following:

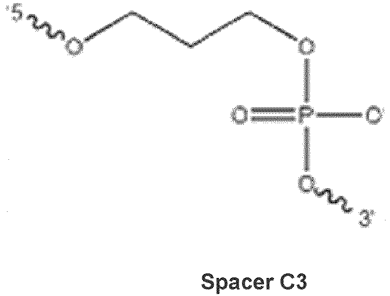

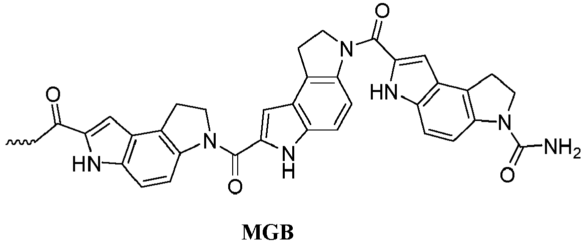

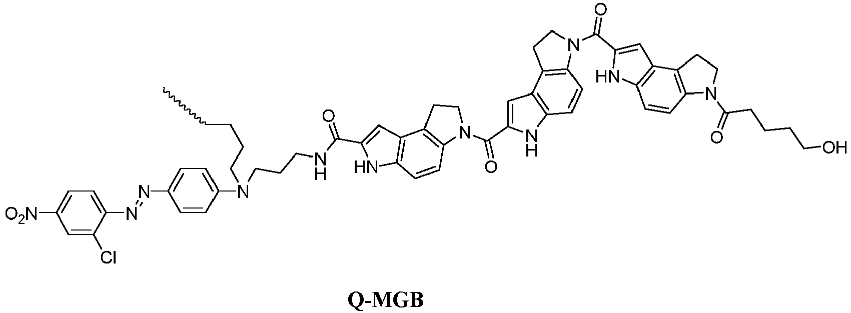

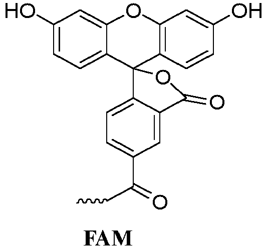

## Claims

1. An oligonucleotide fragment, which is **characterized by** that,
the oligonucleotide fragment comprises a primer region at 3' terminal and a variant recognition region at 5' terminal which are covalently linked, wherein the variant recognition region is covalently linked with a nucleic acid double strand-stabilizing factor or comprises a modifying factor which cannot be hydrolyzed by nuclease, and the variant recognition region forms a hairpin structure with a sequence generated from extension of the primer region.

2. The oligonucleotide fragment according to claim 1, which is **characterized by** that, the modifying factor includes a base analogue, nucleic acid backbone, deoxyribose analogue, peptide nucleic acid or thiophosphate.

3. The oligonucleotide fragment according to claim 1, which is **characterized by** that, the nucleic acid double strand-stabilizing factor includes one of or a combination of more than one of a locked nucleic acid, peptide nucleic acid or DNA minor groove binder/analogue.

4. A method for selective amplification of a variant of a target nucleic acid sequence using an oligonucleotide fragment according to any one of claims 1-3, the method comprising:
(1) hybridizing the primer region in the oligonucleotide fragment with the target nucleic acid sequence in a reaction system, to prepare a template for amplification;
(2) extending 3' terminal of the primer region with nucleic acid polymerase in an amplification system;
(3) when a nucleic acid variant in the sample to be detected is used as the template, the variant recognition region and the sequence generated from extension of the primer region cannot form a stable hairpin structure, thus an amplification reaction proceeds,
when a nucleic acid variant not to be detected in the sample is used as the template, the variant recognition region and the sequence generated from extension of the primer region form a stable hairpin structure, thus the amplification reaction is inhibited.

5. The method for selective amplification of a variant of a target nucleic acid sequence according to claim 4, which is **characterized by** that, a detection method not distinguishing different variants of a nucleic acid is used in combination, including a dye method, hybridization probe detection method, hydrolysis probe detection method, Taqman probe method or molecular beacon method.

6. The method for selective amplification of a variant of a target nucleic acid sequence according to claim 4, which is **characterized by** that, a method distinguishing different variants of a nucleic acid is used in combination, including a sequencing method or high-resolution melting curve method; alternatively, a method of a hybridization probe distinguishing different variants of a nucleic acid, of a hydrolysis probe distinguishing different variants of a nucleic acid, of a Taqman probe distinguishing different variants of a nucleic acid or of a molecular beacon distinguishing different variants of a nucleic acid is used in combination.

7. The method for selective amplification of a variant of a target nucleic acid sequence according to claim 4, which is **characterized by** that, the amplification system is a system which initiates a reaction with the increase of temperature.

8. A kit for detection of a nucleic acid variant, which detects the nucleic acid variant by using an oligonucleotide fragment according to any one of claims 1-3.

9. The kit according to claim 8, which is **characterized by** that, the kit further comprises a reagent required for nucleic acid detection, and the reagent required for nucleic acid detection includes a dye and/or probe.

10. The kit according to claim 8, which is **characterized by** that, the kit further comprises a kit amplification system, and the kit amplification system includes one or a combination of more than one of nucleic acid polymerases, divalent metal ions, nucleotides, salts, buffering agents or cofactors.

## Patentansprüche

1. Oligonukleotidfragment, das **dadurch gekennzeichnet ist, dass** das Oligonukleotidfragment eine Primerregion am 3'-Terminus und eine Variantenerkennungsregion am 5'-Terminus umfasst, die kovalent verknüpft sind, wobei die Variantenerkennungsregion kovalent mit einem Nukleinsäure-Doppelstrang-Stabilisierungsfaktor verknüpft ist oder einen modifizierenden Faktor umfasst, der nicht durch Nuklease hydrolysiert werden kann, und die Variantenerkennungsregion eine Haarnadelstruktur mit einer Sequenz bildet, die durch Verlängerung der Primerregion erzeugt wird.

2. Oligonukleotidfragment nach Anspruch 1, das **dadurch gekennzeichnet ist, dass** der modifizierende Faktor ein Basenanalogon, ein Nukleinsäurerückgrat, ein Desoxyriboseanalogon, eine Peptidnukleinsäure oder ein Thiophosphat beinhaltet.

3. Oligonukleotidfragment nach Anspruch 1, das **dadurch gekennzeichnet ist, dass** der Nukleinsäure-Doppelstrang-Stabilisierungsfaktor eines von oder eine Kombination von mehr als einem von einer fixierten Nukleinsäure, einer Peptidnukleinsäure oder einem DNA-Minor-Groove-Binder/Analogon beinhaltet.

4. Verfahren zur selektiven Amplifikation einer Variante einer Zielnukleinsäuresequenz unter Verwendung eines Oligonukleotidfragments nach einem der Ansprüche 1-3, wobei das Verfahren Folgendes umfasst:
(1) Hybridisieren der Primerregion in dem Oligonukleotidfragment mit der Zielnukleinsäuresequenz in einem Reaktionssystem, um eine Matrize für Amplifikation vorzubereiten;
(2) Verlängern des 3'-Terminus der Primerregion mit Nukleinsäurepolymerase in einem Amplifikationssystem;
(3) wenn eine Nukleinsäurevariante in der Probe, die nachzuweisen ist, als Matrize verwendet wird, die Variantenerkennungsregion und die Sequenz, die durch Verlängerung der Primerregion erzeugt wird, keine stabile Haarnadelstruktur bilden können, sodass eine Amplifikationsreaktion stattfindet,
wenn eine Nukleinsäurevariante, die in der Probe nicht nachzuweisen ist, als Matrize verwendet wird, die Variantenerkennungsregion und die Sequenz, die durch Verlängerung der Primerregion erzeugt wird, eine stabile Haarnadelstruktur bilden, wodurch die Amplifikationsreaktion gehemmt wird.

5. Verfahren zur selektiven Amplifikation einer Variante einer Zielnukleinsäuresequenz nach Anspruch 4, das **dadurch gekennzeichnet ist, dass** ein Nachweisverfahren, das verschiedene Varianten einer Nukleinsäure nicht unterscheidet, in Kombination verwendet wird, beinhaltend ein Farbstoffverfahren, Hybridisierungssondennachweisverfahren, Hydrolysesondennachweisverfahren, Taqman-Sonden-Verfahren oder Molecular-Beacon-Verfahren.

6. Verfahren zur selektiven Amplifikation einer Variante einer Zielnukleinsäuresequenz nach Anspruch 4, das **dadurch gekennzeichnet ist, dass** ein Verfahren, das verschiedene Varianten einer Nukleinsäure unterscheidet, in Kombination verwendet wird, beinhaltend ein Sequenzierungsverfahren oder hochauflösendes Schmelzkurvenverfahren; wobei alternativ ein Verfahren einer Hybridisierungssonde, die verschiedene Varianten einer Nukleinsäure unterscheidet, einer Hydrolysesonde, die verschiedene Varianten einer Nukleinsäure unterscheidet, einer Taqman-Sonde, die verschiedene Varianten einer Nukleinsäure unterscheidet oder eines Molecular-Beacon, der verschiedene Varianten einer Nukleinsäure unterscheidet, in Kombination verwendet wird.

7. Verfahren zur selektiven Amplifikation einer Variante einer Zielnukleinsäuresequenz nach Anspruch 4, das **dadurch gekennzeichnet ist, dass** das Amplifikationssystem ein System ist, das eine Reaktion bei Anstieg der Temperatur initiiert.

8. Kit zum Nachweis einer Nukleinsäurevariante, das die Nukleinsäurevariante unter Verwendung eines Oligonukleotidfragments nach einem der Ansprüche 1-3 nachweist.

9. Kit nach Anspruch 8, **dadurch gekennzeichnet, dass** das Kit ferner ein Reagens umfasst, das für Nukleinsäurenachweis erforderlich ist, und das Reagens, das für Nukleinsäurenachweis erforderlich ist, einen Farbstoff und/oder eine Sonde beinhaltet.

10. Kit nach Anspruch 8, das **dadurch gekennzeichnet ist, dass** das Kit ferner ein Kitamplifikationssystem umfasst und das Kitamplifikationssystem eines von oder eine Kombination von mehr als einem von Nukleinsäurepolymerasen, zweiwertigen Metallionen, Nukleotiden, Salzen und Puffermitteln oder Cofaktoren beinhaltet.

## Revendications

1. Fragment oligonucléotidique, qui est **caractérisé en ce que**,
le fragment oligonucléotidique comprenant une région d'amorce au niveau de l'extrémité 3' et une région de reconnaissance de variant au niveau de l'extrémité 5' qui sont liées de manière covalente, ladite région de reconnaissance de variant étant liée de manière covalente à un facteur de stabilisation de double brin d'acide nucléique ou comprenant un facteur de modification qui ne peut pas être hydrolysé par la nucléase, et ladite région de reconnaissance de variant formant une structure en épingle à cheveux avec une séquence générée à partir de l'extension de la région d'amorce.

2. Fragment oligonucléotidique selon la revendication 1, qui est **caractérisé en ce que** le facteur de modification comprend un analogue de base, un squelette d'acide nucléique, un analogue de désoxyribose, un acide nucléique peptidique ou un thiophosphate.

3. Fragment oligonucléotidique selon la revendication 1, qui est **caractérisé en ce que** le facteur de stabilisation de double brin d'acide nucléique comprend un élément ou une combinaison de plus d'un élément parmi un acide nucléique verrouillé, un acide nucléique peptidique ou un liant/analogue de sillon mineur d'ADN.

4. Méthode permettant l'amplification sélective d'un variant d'une séquence d'acide nucléique cible à l'aide d'un fragment oligonucléotidique selon l'une quelconque des revendications 1-3, le procédé comprenant :
(1) l'hybridation de la région d'amorce dans le fragment oligonucléotidique avec la séquence d'acide nucléique cible dans un système de réaction, afin de préparer une matrice pour l'amplification ;
(2) l'extension de l'extrémité 3' de la région d'amorce avec une polymérase d'acide nucléique dans un système d'amplification ;
(3) lorsqu'un variant d'acide nucléique dans l'échantillon à détecter est utilisé comme matrice, la région de reconnaissance de variant et la séquence générée à partir de l'extension de la région d'amorce ne peuvent pas former une structure en épingle à cheveux stable, ainsi une réaction d'amplification se produit,
lorsqu'un variant d'acide nucléique non à détecter dans l'échantillon est utilisé comme matrice, la région de reconnaissance de variant et la séquence générée à partir de l'extension de la région d'amorce forment une structure en épingle à cheveux stable, ainsi la réaction d'amplification est empêchée.

5. Méthode permettant l'amplification sélective d'un variant d'une séquence d'acide nucléique cible selon la revendication 4, qui est **caractérisée en ce qu'**une méthode de détection ne distinguant pas différents variants d'un acide nucléique est utilisée en combinaison, y compris une méthode de coloration, une méthode de détection par sonde d'hybridation, une méthode de détection par sonde d'hydrolyse, une méthode par sonde Taqman ou une méthode par balise moléculaire.

6. Méthode permettant l'amplification sélective d'un variant d'une séquence d'acide nucléique cible selon la revendication 4, qui est **caractérisée en ce qu'**une méthode distinguant différents variants d'un acide nucléique est utilisé en combinaison, comprenant une méthode de séquençage ou une méthode par courbe de fusion haute résolution ; en variante, une méthode d'une sonde d'hybridation distinguant différents variants d'un acide nucléique, d'une sonde d'hydrolyse distinguant différents variants d'un acide nucléique, d'une sonde Taqman distinguant différents variants d'un acide nucléique ou d'une balise moléculaire distinguant différents variants d'un acide nucléique est utilisée en combinaison.

7. Méthode permettant l'amplification sélective d'un variant d'une séquence d'acide nucléique cible selon la revendication 4, qui est **caractérisée en ce que** le système d'amplification est un système qui initie une réaction avec l'augmentation de la température.

8. Kit permettant la détection d'un variant d'acide nucléique, qui détecte le variant d'acide nucléique à l'aide d'un fragment oligonucléotidique selon l'une quelconque des revendications 1-3.

9. Kit selon la revendication 8, qui est **caractérisé en ce que** le kit comprend en outre un réactif requis pour la détection d'acide nucléique, et le réactif requis pour la détection d'acide nucléique comprend un colorant et/ou une sonde.

10. Kit selon la revendication 8, qui est **caractérisé en ce que** le kit comprend en outre un système d'amplification de kit, et le système d'amplification de kit comprend un élément ou une combinaison de plus d'un élément parmi les polymérases d'acide nucléique, les ions métalliques divalents, les nucléotides, les sels, les agents tampons ou les cofacteurs.
